(19) Europäisches Patentamt
European Patent Office
Office européen des brevets

(11) Numéro de publication : **0 373 993 B1**

(12) # FASCICULE DE BREVET EUROPEEN

(45) Date de publication du fascicule du brevet : **07.04.93 Bulletin 93/14**

(51) Int. Cl.$^5$ : **C07C 31/28,** C07C 29/68, C07C 29/40, C07C 69/24, C07C 67/08

(21) Numéro de dépôt : **89403286.1**

(22) Date de dépôt : **28.11.89**

(54) **Procédé de préparation de composés énoliques et nouveaux produits obtenus.**

(30) Priorité : **02.12.88 FR 8815806**

(43) Date de publication de la demande : **20.06.90 Bulletin 90/25**

(45) Mention de la délivrance du brevet : **07.04.93 Bulletin 93/14**

(84) Etats contractants désignés : **AT BE CH DE ES FR GB GR IT LI LU NL SE**

(56) Documents cités : **Eléments de la technique relevés: néant.**

(73) Titulaire : **SOCIETE NATIONALE ELF AQUITAINE**
**Tour Elf, 2, Place de la Coupole, La Défense 6**
**F-92400 Courbevoie (FR)**

(72) Inventeur : **Cahiez, Gérard**
**68 Rue de Turbigo**
**F-75003 Paris (FR)**
Inventeur : **Figadere, Bruno**
**Paincuit**
**F-28170 Boullay Les Deux Eglises (FR)**
Inventeur : **Tozzolino, Pierre**
**Chemin-Carrerot Serres-Morlaas**
**F-64160 Morlaas (FR)**
Inventeur : **Clery, Patrick**
**3 Passage de l'Union**
**F-75007 Paris (FR)**

(74) Mandataire : **Bertrand, Didier et al**
**Cabinet FEDIT-LORIOT 38, Avenue Hoche**
**F-75008 Paris (FR)**

EP 0 373 993 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention a pour objet un procédé pour la préparation de certains composés énoliques et, plus particulièrement, d'énolates manganeux se prêtant bien à diverses synthèses subséquentes. Elle permet cette obtention dans des bonnes conditions économiques et, de plus, avec une excellente régiosélectivité. L'invention comprend également des énolates manganeux, produits par ce procédé, qui constituent des composés chimiques nouveaux.

Grâce à leur utilité comme intermédiaires de différentes réactions chimiques, des énolates ont été abondamment étudiés et utilisés. Par exemple, FLEMING et coll. ("Synthesis" 1976, 736, et "Chem. Soc. Rev. "1981, 10,83)ont cité leur application à la synthèse de nombreux produits naturels. Ils servent entre autres à la préparation de divers esters, cétones ou aldéhydes utiles en parfumerie ou comme matière première pour la production de médicaments ou d'insecticides.

Il existe de nombreux procédés de préparation d'énolates ; ainsi a-t-on obtenu des éthers d'énols silylés ou des esters d'énols en traitant, par exemple, avec du triméthylchlorosilane, ou avec un anhydride d'acide carboxylique, le produit obtenu par la réaction d'une cétone avec du NaH dans du diméthoxyéthane ou avec du di-isopropylamidure de Li dans ce même solvant. Des éthers d'énols silylés ont également été obtenus en traitant une cétone par une amine tertiaire ou du diazo-1,4 bicyclo(2,2,2)octane en présence de triméthylchlorosilane, dans du diméthylformamide, etc. Dans certains cas, les rendements sont bons, mais avec des cétones dissymétriques, il se forme beaucoup de produit correspondant à l'énolate thermodynamique, tandis que les rendements laissent à désirer dans les rares procédés qui conduisent en majorité au produit correspondant à l'énolate cinétique. En outre, la plupart des techniques antérieures sont d'une exécution délicate, d'autant plus que nombre d'entre elles exigent, pour obtenir l'énolate cinétique, des excès de réactifs (jusqu'à 2,5) et des températures très basses, de l'ordre de -80°C. Ainsi, malgré l'art antérieur existant, le besoin se fait sentir d'un procédé pratique, qui permettrait la préparation économique d'énolates et de leurs dérivés.

La présente invention répond à ce besoin, elle rend possible l'obtention aisée de composés énoliques, à partir de cétones, à des températures peu au-dessus ou au-dessous de 0°C, avec des rendements élevés ou très élevés; le procédé permet la production exclusive ou presque de l' énolate cinétique, lorsque la cétone de départ est dissymétrique. Les énolates obtenus selon l'invention se conservent bien à des températures généralement peu au-dessus ou au-dessous de 0°C.

Le procédé suivant l'invention, qui comprend la réaction d'une cétone avec un composé organo-métallique, est caractérisé en ce que ce composé est un organo-manganeux mixte conduisant à la formation d'un énolate de Mn.

Ce procédé est d'autant plus inattendu que les composés organo-métalliques courants, notamment ceux de Mg ou de Li, donnent - dans les mêmes conditions - le produit résultant d'une addition 1,2 sur le carbonyle de la cétone, c'est-à-dire un alcoolate tertiaire et non l'énolate de la cétone. On a, par exemple, avec RMgX :

(1) ....

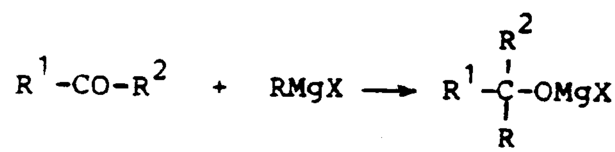

qui, par hydrolyse, conduit à

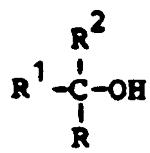

Par contre, suivant l'invention, on fait réagir avec la cétone un organo-manganeux mixte R-MnX donnant à la fois le dérivé manganeux de l'alcool tertiaire

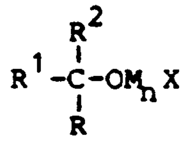

de façon analogue à la réaction (1) ci-dessus, et un énolate formé par le processus :

(2) 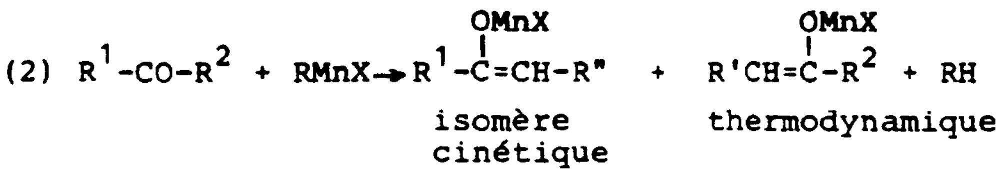


comportant l'arrachement d'un proton avec formation consécutive de RH.

La différence entre les isomères thermodynamique et cinétique apparaît seulement avec les cétones dissymétriques, on la verra donc mieux dans l'exemple de la réaction de R-MnCl avec la propyl éthyl cétone $CH_3CH_2CH_2\text{-}CO\text{-}CH_2CH_3$, qui conduit aux trois composés manganeux :

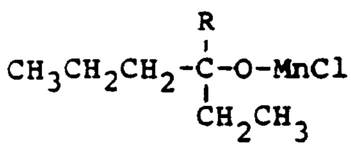 ; 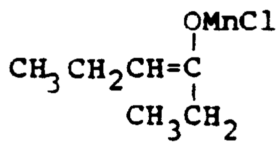 et 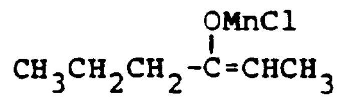

(A) alcoolate tertiaire ; (B) énolate thermodynamique ; (C) énolate cinétique

Les proportions des composés de types A, B et C varient selon la nature de la cétone traitée, celle du solvant, la température, la nature de R, etc. Bien qu'il puisse être utile, dans une certaine industrie, d'obtenir ces trois composés simultanément, il est surtout intéressant de produire au maximum l'un ou l'autre des isomères, soit cinétique, soit thermodynamique, l'isomère cinétique étant le plus difficile à obtenir, par la technique antérieure. Et c'est justement le résultat imprévu que la présente invention permet d'obtenir économiquement.

La préparation des énolates mixtes de manganèse, suivant l'invention, comprend la mise en présence d'une cétone et d'un organo-manganeux mixte dans un solvant approprié, maintenu à une température de -50° à +75°C, durant 1/2 à 20 heures, à l'abri de l'air. Les températures les plus utilisées sont de -10° à +60°C,le temps de la réaction étant le plus souvent de 1 à 3 heures. Les proportions stoechiométriques des réactifs conviennent bien en général, c'est-à-dire qu'on peut employer 1 mole d'organo-manganeux mixte RMnX avec 1 mole de cétone $R^1\text{-}CO\text{-}R^2$, ou 2 moles de RMnX par mole de dicétone.

L'énolate de manganèse formé (B ou/et C vu plus haut) peut être récupéré du milieu réactionnel par évaporation à l'abri de l'air et de l'humidité du solvant ou tout autre moyen connu, pour être ensuite transformé en le dérivé voulu. Cependant, dans la plupart des applications courantes, il est aisé de traiter cet énolate in situ, dans son solvant d'origine, éventuellement en ajoutant un autre solvant ou/et modifiant la température, avant d'introduire un nouveau réactif adéquat. C'est ainsi que l'on peut acyler, silyler, alkyler, halogéner, hydroxyalkyler, etc. le produit dans son milieu réactionnel par l'addition d'un anhydride ou d'un chlorure d'acide, d'un halogénure de trialkylsilyle, d'halogénure d'allyle ou d'alkyle, d'un halogène, d'un aldéhyde, puis de l'eau etc. Le manganèse s'éliminant au cours de telles réactions, on aboutit aux esters d'énols concernés, ou à des β-dicétones, aux dérivés silylés, ou aux cétones correspondantes, alkylées, allylées ou halogénées, etc. Ainsi, le passage par l'énolate mixte de Mn permet-il l'obtention de divers produits résultant de la modification de la cétone initiale.

Le procédé de l'invention s'applique à de nombreuses cétones. Parmi les monocétones conviennent les aliphatiques linéaires, telles que

$$CH_3(CH_2)_n\text{-}CO\text{-}(CH_2)_mCH_3$$

où les nombres n et m, semblables ou différents sont de 0 à 17. D'autre part, dans une ou chacune de leurs chaînes $(CH_2)_n$ et $(CH_2)_m$ il peut y avoir une double ou triple liaison ou/et un substituant alkyle ou aryle. Des cétones similaires portent à la place d'un ou des deux $CH_3$ terminaux un groupe secondaire ou tertiaire, c'est-

à-dire

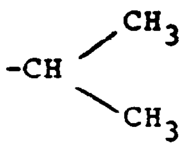

ou

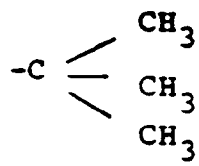

Peuvent également être utilisées des cétones dans lesquelles l'une ou les deux chaînes $CH_3(CH_2)_n$ ou $CH_3(CH_2)_m$ sont remplacées par un cycle phényle, tolyle, xylyle, naphtyle, cyclopentyle , cyclohexyle ou cyclohexényle pouvant porter un ou plusieurs substituants alkyles. Les cétones employées peuvent porter des groupes fonctionnels (halogènes, alcoxy, thioalcoxy, etc).

A titre d'exemples non limitatifs, l'invention peut être réalisée en partant des cétones comme les diéthyl cétone, dipropyl cétone, di-isopropyl cétone, éthyl propyl cétone, éthyl hexyl cétone, éthyl cyclohexyl cétone, éthyl phényl cétone, butyl-cyclopentanone, méthyl-cyclohexanone, hexyl heptyl cétone, butyl dodécyl cétone, acétophénone, etc.

La composition chimique de l'organo-manganeux mixte, RMnX, présente une grande importance pour la réalisation de l'invention. En particulier, le rendement en l'énolate voulu dépend beaucoup de la nature des groupes R et X, dont un choix approprié permet d'atteindre des résultats remarquables.

D'une façon générale, R peut être tout groupe hydrocarboné, aliphatique, cycloaliphatique, arylique amino, ou halogène. Il est cependant préférable qu'il ne porte pas trop de ramifications susceptibles d'exercer de l'empêchement stérique. Conviennent notamment des R constitués par des chaînes alkyles, de préférence méthyle, alcényles ou alcynyles en $C_1$ à $C_{20}$ avec préférence pour $C_1$ à $C_{12}$, notamment des groupes ne possédant pas d'atomes H β, éliminables. R peut également être un cyclopentyle ou cyclohexyle pouvant porter un ou plusieurs substituants d'alkyles en $C_1$ à $C_{12}$.Particulièrement bien conviennent pour R des cycles aryles, en particulier phényle, ou naphtyle, portant éventuellement 1 à 3 substituants alkyles en $C_1$ à $C_{12}$; ainsi les phényle, tolyle, xylyle, mésityle, mono-, di- ou tri-éthylphényle, dipropyl-phényle etc. forment-ils des R intéressants.Le méthyle ou le phényle en R présentent l'avantage de permettre l'obtention de très bons rendements à des températures modérées, notamment entre 0° et 30°C, alors qu'avec des alkyles plus lourds que le méthyle, par exemple le butyle, il faut travailler vers -50°C.

Une grande importance présente la nature du groupe X dans l'organomanganeux RMnX. En principe X peut être tout élément, radical ou groupe susceptible de servir de ligande au complexe du manganèse. Il peut notamment être un halogène, un anion par exemple d'un composé de S, P, B, C, Si, un groupe oxy- ou thiahydrocarboné, un groupe amino- etc. Ainsi, par exemple, X est Cl, Br ou I, $CF_3SO_2$,R'COO, $BF_4$, -OR' ou -SR' (R' étant un alkyle ou aryle), $-NR'_2$ ou -NR' R" (R',R" groupes hydrocarbonés) etc.

Suivant un trait particulier de l'invention, X doit être un groupe basique si l'on cherche à produire le plus possible d'énolate. Il est ainsi possible d'avoir en majeure partie ou exclusivement l'énolate cinétique à partir d'une cétone dissymétrique. Ces réactions présentent également la particularité de conduire principalement à la configuration Z des énolates produits (rapport E/Z 25: 75 à 0:100), ce qui est en général très favorable aux applications industrielles.

En tant que groupes X basiques conviennent bien des groupes amino substitués, du type $-NR'_2$ ou -NR'R" où les radicaux hydrocarbonés R' et R" peuvent être aliphatiques, surtout en $C_1$ à $C_{18}$, cycloaliphatiques ou/et aryliques, notamment phényle pouvant être avantageusement substitués, de préférence avec 1 à 3 alkyles en $C_1$ à $C_{12}$.

Il est préférable qu'au moins un des R' et R" soit un aryle car cela conduit à des rendements bien meilleurs que ne donnent des alkyles seuls. Des phényl méthylamidures du type

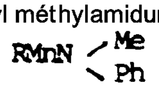

se conservent plusieurs mois à l'ambiante.

On peut voir de ce qui précède que l'invention offre diverses possibilités pour atteindre les résultats voulus avec une cétone donnée : l'homme de l'art peut adopter des groupements R et X appropriés, guidé par les exemples spécifiques se trouvant dans la suite de la présente description.

Un facteur, qui influe également sur les résultats de l'énolisation suivant l'invention, est la nature du solvant. Les principaux solvants utilisables sont des éthers, notamment l'oxyde de diéthyle, le pyrane, le diméthoxy-1,2 éthane et surtout le tétrahydrofurane ; on peut employer, en outre, le diméthylsulfoxyde ou le sulfolane, d'autres solvants étant à la portée de l'homme de l'art qui tiendra compte de ce que les rendements et

la régiosélectivité peuvent varier avec la nature du solvant.

L'utilisation, fort recommandable, de composés organo-manganeux du type RMn-NR′R″, peut être réalisée sous la forme d'une variante procurant une certaine économie. Il s'est avéré qu'au lieu de préparer d'avance le composé RMn-NR′R″, pour l'utiliser dans la réaction (2) en tant que RMnX, on peut employer pour celui-ci un organo-manganeux moins efficace, par exemple RMnCl, mais ajouter au milieu réactionnel de l'amine HNR′R″, susceptible, éventuellement, de former in situ le composé R-Mn-NR′R″. Cela conduit aux mêmes excellents résultats que donne ce composé lorsqu'il a été préparé et mis d'avance dans le milieu réactionnel. Mais il y a plus : on constate ce fait inattendu qu'en ne mettant qu'une partie de l'amine stoechiométriquement requise,par exemple 1/5ème de cette quantité, on obtient un rendement de beaucoup meilleur qu'avec du R-Mn-Cl seul.

Cela conduit à une variante caractérisée en ce que l'énolisation est opérée par la réaction d'une cétone avec un organo-manganeux quelconque, notamment R-Mn-X où X est un halogène, au sein d'un solvant, le milieu étant additionné d'une amine HN-R′R″ en quantité catalytique.

En fait, comme on le voit, d'après les exemples 42 à 46 la quantité d'amine ajoutée peut varier entre environ 5 et 50% de la proportion stoechiométrique par rapport au RMnX présent.

L'invention est illustrée par les exemples non limitatifs qui suivent.

Dans la plupart des exemples on procédait d'abord à la préparation d'un énolate mixte de Mn, à partir d'une cétone ; ensuite on ajoutait au milieu réactionnel un agent acylant, en général l'anhydride propionique $(CH_3CH_2CO)_2O$, de façon à former l'ester propionique correspondant de l'énol ; après séparation de celui-ci, on en déterminait le rendement sur la cétone et le rapport E/Z ; dans le cas des cétones dissymétriques, on évaluait en outre le pourcentage d'isomère cinétique (régiosélectivité)

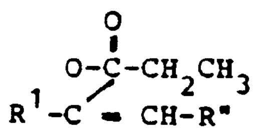

Le mode opératoire consistait à utiliser une solution de 100mmoles de composé organo-manganeux RMnX dans 200 ml de THF ou d'éther entre -10 et 0°C, à laquelle on ajoutait 100 mmoles de cétone, en agitant. On laissait ensuite le mélange revenir à la température ambiante.

Après 2 heures,on refroidissait à -10°C, on ajoutait 200 mmoles d'agent acylant (anhydride propionique) et - sous agitation - on laissait la température remonter à l'ambiante.

Au bout de 2 heures, on soumettait le mélange à l'hydrolyse à -10°C avec 160 ml d'eau. On le filtrait ensuite ou on l'acidifiait (par ClH, $SO_4H_2$) jusqu'à dissolution des sels. La phase aqueuse, décantée, était soumise à l'extraction trois fois avec 100 ml d'éther.

Les phases organiques réunies étaient séchées sur du $MgSO_4$, le solvant en était évaporé sous vide ; le résidu, c'est-à-dire les produits des opérations, étaient isolés et purifiés par distillation.

<u>EXEMPLES 1 à 9</u>

Enolisation de la dipropyl-cétone $C_3H_7$-CO-$C_3H_7$ ($Pr_2CO$) avec différents organo-manganeux butyl-MnX (Bu MnX) à -50°C. Ensuite acylation au moyen de l'anhydride propionique $(C_2H_5CO)_2O$ et dosage des produits obtenus.

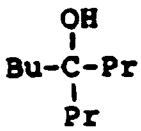

, et

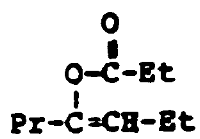

Alcool tertiaire

Ester de l'énol

Les résultats sont réunis au Tableau I ci-après.

TABLEAU I

| Exemple | BuMnX | Solvant | Rendements % : alcool tert. | ester d'énol | cétone récupérée (%) |
|---|---|---|---|---|---|
| 1 | BuMnCl | THF | 75 | 5 à 20 | 0 |
| 2 | BuMnOBu | " | 8 | 34 | 55 |
| 3 | BuMnN(iPr)$_2$ | " | 28 | 47 | 0 |
| 4 | BuMnNPhMe | " | 1 à 2 | 92 | 0 |
| 5 | BuMnNEt$_2$ | $Et_2O$ | 21 | 68 | 3 |
| 6 | BuMnN(iPr)$_2$ | " | 23 | 76 | O |
| 7 | BuMnN 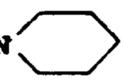 | " | 11 | 40 | 45 |
| 8 | BuMnNPhMe | " | 8 | 90 | 0 |
| 9 | PhMeNMnBr | " | 0 | 70 | 30 |

On peut constater que les meilleurs rendements en ester d'énol sont obtenus avec des BuMnX dont le X est un groupe amino, en particulier N-méthyl phényl amino.

EXEMPLES 10 A 15

Enolisation de la dipropyl-cétone comme dans les exemples 1-9, mais avec des RMnX dont le X est le groupe

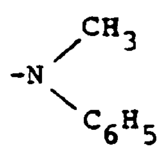 (soit 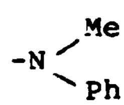),

R variant d'un exemple à l'autre.
Solvant THF.

TABLEAU II

| Ex. n° | R | Température | Rendement % |
|---|---|---|---|
| 10 | Bu | 0°C | 77 |
| 11 | " | -50°C | 92 |
| 12 | Me | 0°C | 93 |
| 13 | Ph | " | 95 |
| 14 | $Me_2C{=}CH$ | " | 92 |
| 15 | PrC≡C- | " | 80 |

Les exemples 12 à 14 indiquent un optimum de rendement en énol lorsqu'on opère à 0°C,R étant un phényle ou méthyle. Les exemples 10 et 11 montrent qu'il est possible d'améliorer le rendement par l'abaissement

de la température d'énolisation lorsque R est un groupe alkyle plus lourd que méthyle.

EXEMPLES 16 à 21

Enolisations *) similaires à celles des exemples précédents, mais à 20°C,avec des organo-manganeux PhMnX où X est un groupe amino changeant d'un exemple à l'autre.

| Ex n° | X | Rendement % |
|---|---|---|
| 16 | 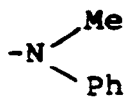 | 95 |
| 17 | 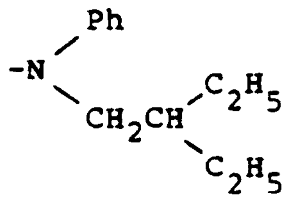 | 94 |
| 18 | 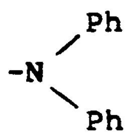 | 92 |
| 19 | 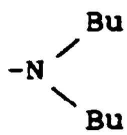 | 40 |
| 20 | 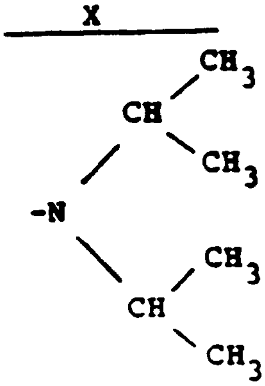 | 40 |
| 21 | 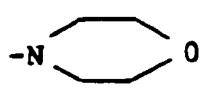 | 23 |

Il se confirme que la présence d'au moins un groupe aryle sur l'azote contribue à une augmentation marquée du rendement en ester d'énol.

EXEMPLES 22 à 28

Enolisation dans l'éther suivant la technique des exemples 1- 9 à -50°C, mais en partant de diverses cétones.

*)Solvant : THF

TABLEAU III

| Ex n° | Cétone | RMnX | Rendements % : alcool tertiaire | ester d'énol |
|---|---|---|---|---|
| 22 | $iPr_2CO$ | $BuMnN(iPr)_2$ | 7 | 65 |
| 23 | PhCOPr | " | 21 | 71 |
| 24 | 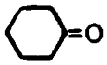 | " | 10 | 60 |
| 25 | $Pr_2CO$ | " | 23 | 76 |
| 26 | " | $BuMnEt_2$ | 21 | 68 |
| 27 | iPrCOBu | " | 3 | 87 |
| 28 | iBuXOHept | " | 6 | 86 |

Les différences de comportement de diverses cétones sont ici plus faciles à observer qu'elles ne le seraient dans des conditions optimales (exemple 4 ou 16), parce que les % d'alcool tertiaire formé dans les exemples 4 ou 16 sont trop faibles pour permettre d'apprécier les différences de comportement.

EXEMPLES 29 à 41

Enolisation à -50°C suivant le mode opératoire des exemples précédents,partant de différentes cétones avec différents organo-manganeux mixtes, tantôt dans de l'éther, tantôt dans du tétrahydrofuranne ; différents anhydrides sont ensuite employés pour acyler l'énolate formé ; en plus du rendement en ester d'énol correspondant à l'énolate cinétique, on détermine la configuration stéréochimique cis/trans de cet ester (rapport E/Z).

TABLEAU IV

| Ex. n° | Cétone | BuMnX | Solvant | $(RCO)_2O$ | Cinétique Rendement (%) | Cinétique E/Z | Thermo Rendement |
|---|---|---|---|---|---|---|---|
| 29 | $(CH_3)_2CH-CO(CH_2)_3-CH_3$ | $BuMnNEt_2$ | $Et_2O$ | $(EtCO)_2O$ | 87 | 0/100 | 0 |
| 30 | $(CH_3)_2CH-CH_2-CO(CH_2)_6CH_3$ | " | " | " | 86 | 10/90 | 2 |
| 31 | 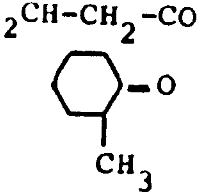 | BuMnNPhMe | " | " | 96 | - | 0 |
| 32 | EtCOEt | " | " | " | 88 | 20/80 | - |
| 33 | " | " | THF | " | 92 | 0/100 | - |
| 34 | PrCOPr | " | $Et_2O$ | " | 90 | 20/80 | - |
| 35 | " | " | THF | " | 91 | 0/100 | - |
| 36 | " | " | $Et_2O$ | $(iPrCO)_2O$ | 83 | 25/75 | - |
| 37 | " | " | " | $(BuCO)_2O$ | 88 | 20/80 | - |
| 38 | PrCOPr | $BuMnN(iPr)_2$ | " | $(EtCO)_2O$ | 76 | 20/80 | - |
| 39 | iPrCOiPr | " | " | " | 65 | - | - |
| 40 | PhCOPr | BuMnNPhMe | " | " | 80 | 0/100 | - |
| 41 | 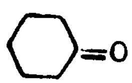 | $BuMnN(iPr)_2$ | " | " | 60 | - | - |

Il est frappant de voir que le rapport E/Z est toujours compris entre 25/75 et 0/100 (soit 1/3 à 0), alors que, dans la technique antérieure, avec des rendements de 78 à 100% on avait des E/Z de 95/5 à 99,5/0,5 (R.E. IRELAND et coll. J. Amer. Chem. Soc. 1976, 2868 ; E.I. NAKAMURA et coll. Tetrahedron Letters 1978, 2079). Deux auteurs auraient réussi, par l'emploi du tétraméthyl-2,2,6,6 pipéridure de Li (LiTMP) pour l'énolisation, d'obtenir des rapports E/Z de 16/84 et 5/95, mais alors les rendements ne dépassaient pas 70% (E.I. NAKAMURA cité plus haut et Z.A. FATAFTAH et coll. G. Am. Chem. Soc. 1980, 3959).

EXEMPLES 42-46

Enolisation suivant une variante utilisant une amine comme catalyseur
Les grandes lignes du mode opératoire sont celles des exemples 1 à 9 avec, comme organomanganeux, le chlorure de phényl manganèse

$$C_6H_5\text{-}MnCl$$

dont 100 mmoles sont mises à réagir avec 100 mmoles de dipropyl-cétone $C_3H_7\text{-}CO\text{-}C_3H_7$, dans 200 ml de THF, à 0°C, pendant 30 minutes, le milieu étant préalablement additionné d'une quantité d'amine

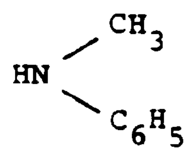

indiqué dans le tableau des résultats V ci-après. Comme pour les exemples précédents, le milieu réactionnel, contenant l'énolate mixte de Mn formé, était traité par de l'anhydride propionique et l'on déterminait le rendement en ester d'énol obtenu.

Le tableau des résultats, qui suit, indique les nombres de moles d'amine et de Ph-MnCl utilisées pour 100 moles de cétone.

TABLEAU V

| Exemple n° | Moles PhMnCl | Moles Amine | | Rendement % |
|---|---|---|---|---|
| 42 | 107,5 | 7,5 | soit 6,9% | 72 |
| 43 | 120 | 20 | " 16,7 | 85-88 |
| 44 | 150 | 50 | " 33,3 | 87 |
| 45 | 200 | 100 | " 50,0 | 95 |
| 46 | 100 | 0 | 0 | 64 |

EXEMPLE 47

Enolisation suivant l'exemple 43 au sein d'un solvant spécial, formé de 3 volumes de THF avec 1 volume de sulfolane

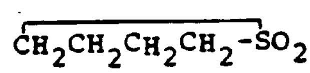

(tétraméthylène sulfone). On obtient alors, avec 20% d'amine, un rendement de 94%.

EXEMPLES 48 à 54

Application d'énolates manganeux mixtes à l'alkylation. Dans les exemples précédents, la solution d'énolate de Mn, obtenue suivant l'invention, était traitée par un anhydride d'acide, ce qui conduisait à un ester d'énol.

Ici, le traitement subséquent est effectué par l'adjonction à la solution dans du THF d'un halogénure organique $R^3X$. 2 moles d'un $R^3X$ sont donc ajoutées à 1 mole d'énolate de Mn ; on opère à la température ambiante, pendant 2 heures, après quoi le produit formé est isolé. On obtient ainsi une cétone homologue de celle, $R^1COR^2$, qui a servi à la formation del'énolate manganeux, suivant les réactions:

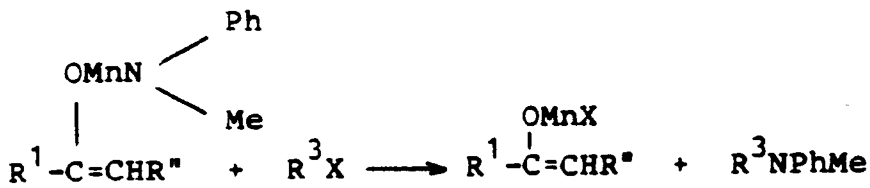

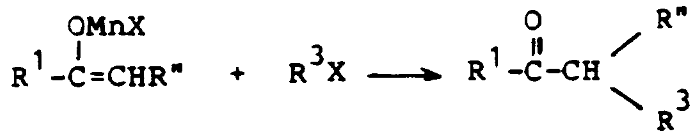

nouvelle cétone.

Le Tableau VI ci-après indique les rendements en la nouvelle cétone, obtenus par rapport à celle qui a servi à la préparation de l'organo-manganeux.

**TABLEAU VI**

| Ex n° | Cétone initiale | Solvant | $R^3X$ | Rendement % en cétone nouvelle |
|---|---|---|---|---|
| 48 | Pr-CO-Pr | THF | $ICH_3$ | 76 |
| 49 | " | " | $IC_4H_9$ | 55 |
| 50 | " | " +DMSO | " | 62 |
| 51 | " | THF | $PhCH_2Br$ | 75 |
| 52 | Pr-CO-Pr | THF/DMSO | Bromure d'allyle ($CH_2=CH-CH_2Br$) | 91 |
| 53 | Pr-CO-Pr | THF/ sulfolane | " " | 98 |
| 54 | Bu-CO-Bu | THF | $BrCH_2COOEt$ | 88 |

EXEMPLES 55-63

Application des énolates manganeux mixtes à l'obtention d'éthers d'énols silylés. Opération à -50°C. De façon analogue aux exemples précédents, une fois préparée, la solution d'énolate manganeux est additionnée de 1,3 mole de chlorure de triméthyl-silyle $(CH_3)_3SiCl$ par groupe -MnX présent sous la forme de

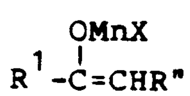

et

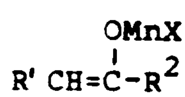

(isomère thermo-dynamique) (cinétique)

En 2 heures à la température ambiante, on obtient les deux isomères correspondants de l'éther silylé, lorsque la cétone de départ, ayant servi à la production de l'énolate manganeux, est dissymétrique. On a ainsi :

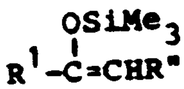

(thermodynamique)

et

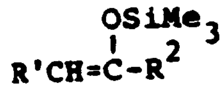

(cinétique)

A partir de diverses cétones, et différents organo-manganeux, on a obtenu les éthers d'énols silylés avec les rendements et les proportions d'isomères suivants.

TABLEAU VII

| Ex. n° | Cétone initiale | Bu-MnX | Rapport ciné/thermo | Rendement % global | Rapport Z/E (isomère cinétique) |
|---|---|---|---|---|---|
| 55 | PrCOPr | Bu-MnNPhMe | - | 88 | 100/0 |
| 56 | iPrCOBu | " | 100/0 | 70 | 100/0 |
| 57 | PhCOPr | " | - | 89 | 100/0 |
| 58 | iBuCOHept | " | 100/0 | 93 | 98/2 |
| 59 | $PhCH_2COPr$ | " | 100/0 | 90 | 100/0 |
| 60 | BuCOEt | " | 66/34 | 90 | - |
| 61 | " | $Bu-MnN(iPr)_2$ | 53/47 | 90 | - |
| 62 | " | $Bu-MnNPh_2$ | 55/45 | 79 | - |
| 63 | HeptCOEt | Bu-MnNPhMe | 58/42 | 89 | - |

Nota : On trouve ici des rapports Z/E encore plus remarquables qu'au Tableau IV (voir page 15, lignes 1-11).

EXEMPLES 64 à 68 - Préparation et Silylation d'énolates manganeux entre -10° et 0°C dans du THF. L'organo-manganeux utilisé est

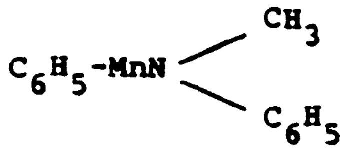

la silylation est effectuée avec 1,2 équivalent $(CH_3)_3SiCl$ par rapport à la cétone.

Selon la cétone employée, les rendements en éther silylé sont :

| Exemple n° | Cétone | Rendement % |
|---|---|---|
| 64 | Pr-CO-Pr | 80 |
| 65 | 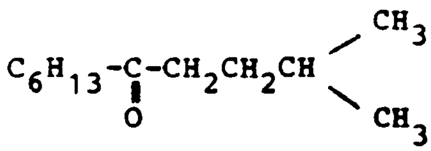 | 82 |
| 66 | 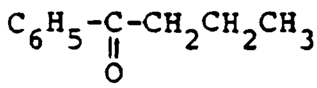 | 86 |
| 67 | 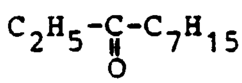 | 86 |
| 68 | 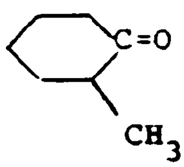 | 88 |

EXEMPLES 69 et 70

Ces exemples montrent que, dans le cadre de la présente invention, lorsque la réaction d'énolisation est concurrencée par la réaction d'addition 1-2, il est possible d'améliorer le rendement en énolate par une élévation de la température. Les matières de départ sont la méthyl-3 cyclohexanone et le méthyl phényl amino phényl-manganèse ; l'énolisation est effectuée dans du THF pendant 1 heure à la température T indiquée plus loin ; elle est suivie d'une silylation avec du $(CH_3)_3$ Si Cl, à 20°C, durant 30 minutes. On obtient alors les trois composés suivants en les proportions qui varient avec la température T d'énolisation. Voici les rendements obtenus :

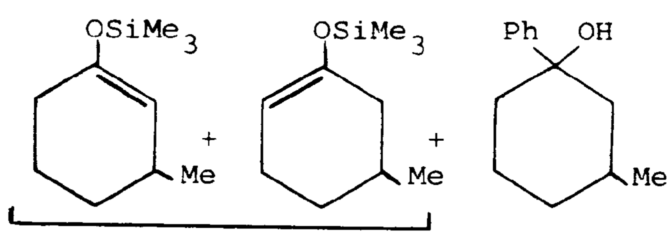

| | | |
|---|---|---|
| Exemple 69---T=-10°C | 15 % | 75 % |
| " 70---T=+60°C | 63 % | 20 % |

Ainsi a-t-on considérablement amélioré le rendement en composés silylés par l'augmentation de la température à 60°C.

EXEMPLES 71-76

Des énolisations d'une heure, suivies de silylations de 30 mn, sont effectuées comme dans les exemples 69-70, mais tout à 20°C. Les composés manganeux, employés, RMnX sont tous des amidures, X étant

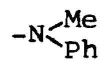

mais R est tantôt un groupe hydrocarboné, notamment Ph, tantôt un halogène.

Dans les exemples 71 à 73 ou part de la méthyl-2 cyclohexanone et l'on détermine le rendement en le triméthylsilyle obtenu

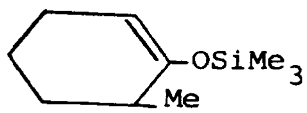 -----(A)

tandis que les exemples 74-76 portent sur la n-hexyl isopropyl cétone que l'on transforme en le composé silylé correspondant,

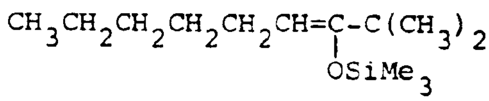 ---(B)

Les résultats sont rapportés ci-après.

| Exemples | R | solvant | Rendement % | |
|---|---|---|---|---|
| 71 | Ph | THF | A | 88 |
| 72 | Cl | THF | " | 86 |
| 73 | Cl | THF/sulfolane | " | 85 |
| 74 | Ph | THF | B | 90 |
| 75 | Cl | THF | " | 90 |
| 76 | Cl | THF/sulfolane | " | 89 |

Nota : le mélange THF/sulfolane est celui de l'exemple 47.

Ces résultats comparatifs indiquent qu'un halogène au lieu d'un radical hydrocarboné, en R du composé manganeux, ne modifie pratiquement pas le rendement, alors qu'il apporte un avantage d'économie et de stabilité.

EXEMPLES 77-78

L'amidure RMnX des exemples 71 et 74, soit

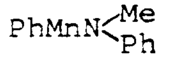

se prépare à partir de

$$PhLi + MnCl_2 + LiN{<}^{Me}_{Ph}$$

ou de

$$2PhLi + MnCl_2 + HN{<}^{Me}_{Ph}$$

tandis qu'il suffit de

$$MnCl_2 + LiN{<}^{Me}_{Ph}$$

ou de

$$RLi + MnCl_2 + HN{<}^{Me}_{Ph}$$

pour obtenir

$$ClMnN{<}^{Me}_{Ph} \;;$$

la préparation de ce dernier a donc lieu avec une économie de composé organométallique. En outre, dans la préparation d'énolates, ce second amidure, dont le R est un Cl, apporte une économie de réactif, par ce que l'énolate est du type

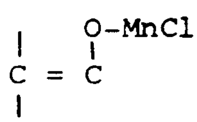 au lieu de 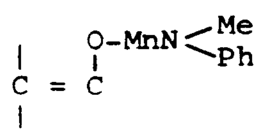

A titre d'exemple, on donne ci-après les résultats de la préparation de benzyl-propyl cétone à partir de la dipropyl cétone, par énolisation avec un composé manganeux

$$RMnN{<}^{Me}_{Ph}$$

à 20°C, pendant 1 heure, puis réaction avec du bromure de benzyle $PhCH_2Br$ à 20°C en 3 heures.

Dans les deux cas décrits ici on obtient la nouvelle cétone

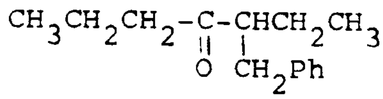

avec les rendements indiqués ci-après.

| Exemples | R | Equivalents $PhCH_2Br$ | Solvant | Rendement % |
|---|---|---|---|---|
| 77 | Ph | 2,1 | THP | 75% + $PhCH_2N{<}^{Me}_{Ph}$ |
| 78 | Cl | 1,05 | THF/sulfolane | 83% - |

Ainsi, non seulement le rendement est meilleur avec l'amidure dont R est Cl, mais on consomme deux fois moins de réactif (ici $PhCH_2Br$). On constate en outre que cet amidure est plus stable que celui de Ph et il permet de travailler en milieu plus concentré.

## Revendications

**Revendications pour les Etats contractants suivants : AT, BE, CH, DE, FR, GB, IT, LI, LU, NL, SE**

**1.** Procédé de préparation d'un dérivé énolique à partir d'une cétone,par l'action d'un composé organo-métallique, caractérisé en ce que ce composé est un organo-manganeux mixte conduisant à la formation d'un énolate de Mn.

**2.** Procédé suivant la revendication 1, caractérisé en ce que dans l'organo-manganeux mixte R-Mn-X, R désigne un groupe hydrocarboné, ou un groupe amino ou halogène et X tout groupe susceptible de constituer un ligande d'un complexe du manganèse et, en particulier, un halogène, un anion dérivé, de S, P, B, C ou Si, un groupe oxy- ou thio-hydrocarboné, ou un groupe amino.

**3.** Procédé suivant la revendication 2, caractérisé en ce que R est une chaîne alkyle , en $C_1$ à $C_{20}$, de préférence en $C_1$ à $C_{12}$, alcényle ou alcynyle en $C_2$ à $C_{20}$, de préférence en $C_2$ à $C_{12}$, ou un cycloalkyle, notamment cyclopentyle ou cyclohexyle pouvant porter un ou plusieurs substituants alkyles en $C_1$ à $C_{12}$.

**4.** Procédé suivant la revendication3,caractérisé en ce que R est méthyle.

**5.** Procédé suivant la revendication 2, caractérisé en ce que R est un aryle, en particulier phényle, ou naphtyle, pouvant porter 1 à 3 substituants alkyles en $C_1$ à $C_{12}$

**6.** Procédé suivant une des revendications 1 à 5, caractérisé en ce que X est un groupe basique, en particulier -NR'R", où R' et R" sont des groupes hydrocarbonés semblables ou différents, de préférence aliphatiques en $C_1$ à $C_{18}$ ou/et aryliques pouvant être substitués avec 1 à 3 alkyles en $C_1$ à $C_6$.

**7.** Procédé suivant la revendication 6, caractérisé en ce que X est un groupe -NR'R" où R' est un alkyle en $C_1$ à $C_6$ ; R" étant un aryle, ou bien à la fois R' et R" sont des aryles.

**8.** Procédé suivant une des revendications précédentes, dans lequel les réactifs sont préparés dans un solvant, plus particulièrement dans un éther, surtout le tétrahydrofuranne.

**9.** Procédé suivant une des revendications précédentes, caractérisé en ce que la réaction a lieu entre -50° et +75°C, et surtout entre -10° et +60°C.

**10.** Procédé suivant une des revendications précédentes, caractérisé en ce que dans RMnX, X est un halogène ou un anion, et la solution des réactifs est additionnée d'une amine HNR'R" à raison de 5 à 50 moles pour 100 moles de RMnX présentes.

**11.** Procédé suivant la revendication 10, caractérisé en ce que la réaction est effectuée dans un mélange THF/sulfolane.

**12.** Nouveau produit chimique constitué par un énolate de Mn mixte.

13. Procédé suivant la revendication 12, caractérisé par les formules

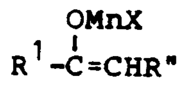 et 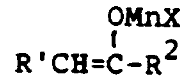

où $R^1$, $R^2$, $R'$ et $R''$ sont des groupes hydrocarbonés aliphatiques en $C_1$ à $C_{17}$ ou/et aryliques, X étant défini dans la revendication 2.

14. Produit suivant la revendication 13, caractérisé en ce que X est un groupe basique comme défini dans une des revendications 6 et 7.

15. Application d'un énolate de Mn mixte, suivant une des revendications 12 à 14, à la préparation d'un dérivé d'énol ou de cétone, caractérisé en ce que l'énolate mixte de Mn est soumis à l'acylation, à l'alkylation, à l'alcénylation, à la silylation, à l'halogénation ou à l'hydroxyalkylation, avec élimination du Mn.

**Revendications pour les Etats contractants suivants : ES, GR**

1. Procédé de préparation d'un dérivé énolique à partir d'une cétone,par l'action d'un composé organo-métallique, caractérisé en ce que ce composé est un organo-manganeux mixte conduisant à la formation d'un énolate de Mn.

2. Procédé suivant la revendication 1, caractérisé en ce que dans l'organo-manganeux mixte R-Mn-X, R désigne un groupe hydrocarboné, ou un groupe amino ou halogène et X tout groupe susceptible de constituer un ligand d'un complexe du maganèse et, en particulier, un halogène, un anion dérivé, de S, P, B, C ou Si, un groupe oxy- ou thio-hydrocarboné, ou un groupe amino.

3. Procédé suivant la revendication 2, caractérisé en ce que R est une chaîne alkyle, en $C_1$ à $C_{20}$, de préférence en $C_1$ à $C_{12}$, alcényle ou alcynyle en $C_2$ à $C_{20}$, de préférence en $C_2$ à $C_{12}$, ou un cycloalkyle, notamment cyclopentyle ou cyclohexyle pouvant porter un ou plusieurs substituants alkyles en $C_1$ à $C_{12}$.

4. Procédé suivant la revendication3,caractérisé en ce que R est méthyle.

5. Procédé suivant la revendication 2, caractérisé en ce que R est un aryle, en particulier phényle, ou naphtyle, pouvant porter 1 à 3 substituants alkyles en $C_1$ à $C_{12}$

6. Procédé suivant une des revendications 1 à 5, caractérisé en ce que X est un groupe basique, en particulier -NR'R'', où R' et R'' sont des groupes hydrocarbonés semblables ou différents, de préférence aliphatiques en $C_1$ à $C_{18}$ ou/et aryliques pouvant être substitués avec 1 à 3 alkyles en $C_1$ à $C_6$.

7. Procédé suivant la revendication 6, caractérisé en ce que X est un groupe -NR'R'' ou R' est un alkyle en $C_1$ à $C_6$ ; R'' étant un aryle, ou bien à la fois R' et R'' sont des aryles.

8. Procédé suivant une des revendications précédentes, dans lequel les réactifs sont préparés dans un solvant, plus particulièrement dans un éther, surtout le tétrahydrofuranne.

9. Procédé suivant une des revendications précédentes, caractérisé en ce que la réaction a lieu entre -50° et +75°C, et surtout entre -10° et +60°C.

10. Procédé suivant une des revendications précédentes, caractérisé en ce que dans RMnX, X est un halogène ou un anion, et la solution des réactifs est additionnée d'une amine HNR'R'' à raison de 5 à 50 moles pour 100 moles de RMnX présentes.

11. Procédé suivant la revendication 10, caractérisé en ce que la réaction est effectuée dans un mélange THF/sulfolane.

12. Procédé suivant la revendication 2, caractérisé en ce que l'énolate est de la forme

$$R^1-\overset{\displaystyle OMnX}{\overset{|}{C}}=CHR'' \qquad \text{ou/et} \qquad R'CH=\overset{\displaystyle OMnX}{\overset{|}{C}}-R^2$$

où $R^1$, $R^2$, R′ et R″ sont des groupes hydrocarbonés aliphatiques en $C_1$ à $C_{17}$ ou/et aryliques.

**13.** Procédé suivant une des revendications 2 à 11, caractérisé en ce que X est un groupe hydrocarboné, notamment -NR′R″, lorsque R est halogène.

**14.** Application du procédé suivant une des revendications 1 à 13, à la préparation d'un dérivé d'énol ou de cétone, caractérisé en ce que l'énolate mixte de Mn est soumis à l'acylation, à l'alkylation, à l'alcénylation, à la silylation, à l'halogénation ou à l'hydroxyalkylation, avec élimination du Mn.

## Patentansprüche

### Patentansprüche für folgende Vertragsstaaten : AT, BE, CH, DE, FR, GB, IT, LI, LU, NL, SE

**1.** Verfahren zur Herstellung eines Enolderivates, ausgehend von einem Keton, mittels einer Organo-Metallverbindung, dadurch gekennzeichnet, daß diese Verbindung ein Organo-Manganverbund ist, der zur Bildung eines Mn-Enolates führt.

**2.** Verfahren nach Anspruch 1, dadurch gekennzeichnet, daß in dem Organo-Manganverbund R-Mn-X R eine Kohlenwasserstoffgruppe oder eine Amino- oder Halogengruppe darstellt und X für sämtliche Gruppen steht, die geeignet sind, einen Liganden eines Mangankomplexes zu bilden, insbesondere für Halogen, ein Anion, das sich von S, P, B, C oder Si ableitet, eine Oxy- oder Thiokohlenwasserstoffgruppe oder eine Aminogruppe.

**3.** Verfahren nach Anspruch 2, dadurch gekennzeichnet, daß R für eine $C_1$ bis $C_{20}$-Alkylkette, vorzugsweise eine $C_1$ bis $C_{12}$-Alkylkette, für $C_2$ bis $C_{20}$-Alkenyl oder -Alkynyl, vorzugsweise $C_1$ bis $C_{12}$-Alkenyl oder -Alkynyl, oder Cycloalkyl, insbesondere Cyclopentyl oder Cyclohexyl, die einen oder mehrere $C_1$ bis $C_{12}$-Alkylsubstituenten tragen können, steht.

**4.** Verfahren nach Anspruch 3, dadurch gekennzeichnet, daß R Methyl bedeutet.

**5.** Verfahren nach Anspruch 2, dadurch gekennzeichnet, daß R Aryl, insbesondere Phenyl oder Naphthyl, darstellt, das 1 bis 3 $C_1$ bis $C_{12}$-Alkylsubstituenten tragen kann.

**6.** Verfahren nach einem der Ansprüche 1 bis 5, dadurch gekennzeichnet, daß X eine basische Gruppe ist, insbesondere -NR′R″, worin R′ und R″ gleiche oder verschiedene Kohlenwasserstoffgruppen, vorzugsweise aliphatische $C_1$ bis $C_{18}$-Kohlenwasserstoffgruppen, oder/und arylische, die mit 1 bis 3 $C_1$ bis $C_6$-Alkylen substituiert sein können, darstellen.

**7.** Verfahren nach Anspruch 6, dadurch gekennzeichnet, daß X für eine NR′R″-Gruppe steht, worin R′ $C_1$ bis $C_6$-Alkyl bedeutet, wobei R″ Aryl darstellt oder auch R′ und R″ gleichzeitig Aryl darstellen.

**8.** Verfahren nach einem der vorausgegangenen Ansprüche, bei dem die Reagentien in einem Lösungsmittel, insbesondere in einem Ether, insbesondere Tetrahydrofuran, vorbereitet werden.

**9.** Verfahren nach einem vorausgegangen Ansprüche, dadurch gekennzeichnet, daß die Reaktion zwischen -50° C und +75 ° C, insbesondere zwischen -10° C und +60° C, stattfindet.

**10.** Verfahren nach einem der vorausgegangenen Ansprüche, dadurch gekennzeichnet, daß in RMnX X für Halogen oder ein Anion steht und zu der Lösung der Reagentien ein Amin HNR′R″ in einem Verhältnis von 5 bis 50 Mol auf 100 vorhandene Mol RMnX zugegeben wird.

**11.** Verfahren nach Anspruch 10, dadurch gekennzeichnet, daß Die Umsetzung in einem Gemisch aus THF/Sulfolan durchgeführt wird.

**12.** Neues chemisches Produkt, das einen Mn-Enolatverbund umfaßt.

**13.** Produkt nach Anspruch 12, gekennzeichnet durch die Formeln

```
   OmnX                     OMnX
   |                        |
R1-C=CHR"      und     R'CH=C-R2
```

worin $R^1$, $R^2$, R′ und R″ aliphatische $C_1$ bis $C_{17}$-Kohlenwasserstoffgruppen, oder/und Aryl-Kohlenwasserstoffgruppen darstellen, wobei X die in Anspruch 2 gegebene Definition besitzt.

**14.** Produkt nach Anspruch 13, dadurch gekennzeichnet, daß X, wie in einem der Ansprüche 6 und 7 definiert, für eine basische Gruppe steht.

**15.** Verwendung eines Mn-Enolatverbundes nach einem der Ansprüche 12 bis 14 zur Herstellung eines Enol- oder Ketonderivates, dadurch gekennzeichnet daß, der Mn-Enolatverbund einer Acylierung, Alkylierung, Alkenylierung, Silylierung, Halogenierung oder Hydroxyalkylierung unter Elimierung von Mn unterzogen wird.

**Patentansprüche für folgende Vertragsstaaten : ES, GR**

**1.** Verfahren zur Herstellung eines Enolderivates, ausgehend von einem Keton, mittels einer Organo-Metallverbindung, dadurch gekennzeichnet, daß diese Verbindung ein Organo-Manganverbund ist, der zur Bildung eines Mn-Enolates führt.

**2.** Verfahren nach Anspruch 1, dadurch gekennzeichnet, daß in dem Organo-Manganverbund R-Mn-X R eine Kohlenwasserstoffgruppe oder eine Amino- oder Halogengruppe darstellt und X für sämtliche Gruppen steht, die geeignet sind, einen Liganden eines Mangankomplexes zu bilden, insbesondere für Halogen, ein Anion, das sich von S, P, B, C oder Si ableitet, eine Oxy- oder Thiokohlenwasserstoffgruppe oder eine Aminogruppe.

**3.** Verfahren nach Anspruch 2, dadurch gekennzeichnet, daß R für eine $C_1$ bis $C_{20}$-Alkylkette, vorzugsweise eine $C_1$ bis $C_{12}$-Alkylkette, für $C_2$ bis $C_{20}$-Alkenyl oder -Alkynyl, vorzugsweise $C_1$ bis $C_{12}$-Alkenyl oder -Alkynyl, oder Cycloalkyl, insbesondere Cyclopentyl oder Cyclohexyl, die einen oder mehrere $C_1$ bis $C_{12}$-Alkylsubstituenten tragen können, steht.

**4.** Verfahren nach Anspruch 3, dadurch gekennzeichnet, daß R Methyl bedeutet.

**5.** Verfahren nach Anspruch 2, dadurch gekennzeichnet, daß R Aryl, insbesondere Phenyl oder Naphthyl, darstellt, das 1 bis 3 $C_1$ bis $C_{12}$-Alkylsubstituenten tragen kann.

**6.** Verfahren nach einem der Ansprüche 1 bis 5, dadurch gekennzeichnet, daß X eine basische Gruppe ist, insbesondere -NR′R″, worin R′ und R″ gleiche oder verschiedene Kohlenwasserstoffgruppen, vorzugsweise aliphatische $C_1$ bis $C_{18}$-Kohlenwasserstoffgruppen, oder/und arylische, die mit 1 bis 3 $C_1$ bis $C_6$-Alkylen substituiert sein können, darstellen.

**7.** Verfahren nach Anspruch 6, dadurch gekennzeichnet, daß X für eine NR′R″-Gruppe steht, worin R′ $C_1$ bis $C_6$-Alkyl bedeutet, wobei R″ Aryl darstellt oder auch R′ und R″ gleichzeitig Aryl darstellen.

**8.** Verfahren nach einem der vorausgegangenen Ansprüche, bei dem die Reagentien in einem Lösungsmittel, insbesondere in einem Ether, insbesondere Tetrahydrofuran, vorbereitet werden.

**9.** Verfahren nach einem vorausgegangen Ansprüche, dadurch gekennzeichnet, daß die Reaktion zwischen -50° C und +75 ° C, insbesondere zwischen -10° C und +60° C, stattfindet.

**10.** Verfahren nach einem der vorausgegangenen Ansprüche, dadurch gekennzeichnet, daß in RMnX X für Halogen oder ein Anion steht und zu der Lösung der Reagentien ein Amin HNR′R″ in einem Verhältnis von 5 bis 50 Mol auf 100 vorhandene Mol RMnX zugegeben wird.

**11.** Verfahren nach Anspruch 10, dadurch gekennzeichnet, daß Die Umsetzung in einem Gemisch aus THF/Sulfolan durchgeführt wird.

12. Verfahren nach Anspruch 2, dadurch gekennzeichnet, daß das Enol die folgende Formel aufweist

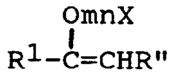 und 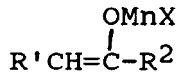

worin $R^1$, $R^2$, $R'$ und $R''$ aliphatische $C_1$ bis $C_{17}$-Kohlenwasserstoffgruppen oder/und Aryl-Kohlenwasserstoffgruppen darstellen.

13. Verfahren nach einem der Ansprüche 2 bis 11, dadurch gekennzeichnet, daß X eine Kohlenwasserstoffgruppe, insbesondere -NR′R″, bedeutet, wenn R für Halogen steht.

14. Anwendung des Verfahrens nach einem der Ansprüche 1 bis 13 zur Herstellung eines Enol- oder Ketonderivates, dadurch gekennzeichnet, daß der Mn-Enolatverbund einer Acylierung, Alkylierung, Alkenylierung, Silylierung, Halogenierung oder Hydroxyalkylierung unter Elimierung von Mn unterzogen wird.

## Claims

### Claims for the following Contracting States : AT, BE, CH, DE, FR, GB, IT, LI, LU, NL, SE

1. Process for preparation of an enolic derivative from a ketone, by the action of an organometallic compound, characterised in that the compound is a mixed organomanganous compound leading to the formation of an enolate of Mn.

2. Process according to claim 1, characterised in that in the mixed organomanganous compound R-Mn-X, R denotes a hydrocarbon group, or an amino group or halogen, and X any group able to constitute a ligand of a manganese complex and, in particular, a halogen, an anion derived from S, P, B, C or Si, an oxy- or thio-hydrocarbon group, or an amino group.

3. Process according to claim 2, characterised in that R is a $C_1$ to $C_{20}$ alkyl chain, preferably $C_1$ to $C_{12}$, a $C_2$ to $C_{20}$, preferably $C_2$ to $C_{12}$, alkenyl or alkynyl group, or a cycloalkyl, particularly cyclopentyl or cyclohexyl, group, possibly carrying one or more $C_1$ to $C_{12}$ alkyl substituents.

4. Process according to claim 3, characterised in that R is methyl.

5. Process according to claim 2, characterised in that R is an aryl, in particular phenyl, or naphthyl, possibly carrying 1 to 3 $C_1$ to $C_{12}$ alkyl substituents.

6. Process according to one of claims 1 to 5, characterised in that X is a basic group, in particular -NR′R″, where R′ and R″ are the same or different hydrocarbon groups, preferably $C_1$ to $C_{18}$ aliphatic and/or arylic groups possibly being substituted with 1 to 3 $C_1$ to $C_6$ alkyl groups.

7. Process according to claim 6, characterised in that X is an -NR′R″ group, where R is a $C_1$ to $C_6$ alkyl; R″ being an aryl group, or else, at the same time, R′ and R″ are aryl groups.

8. Process according to one of the preceding claims, in which the reagents are provided in a solvent, more particularly in an ether, most particularly tetrahydrofuran.

9. Process according to one of the preceding claims, characterised in that the reaction takes place between -50° and +75°C, particularly between -10° and +60°C.

10. Process according to one of the preceding claims, characterised in that, in RMnX, X is a halogen or an anion, and the solution of the reagents is mixed with an amine HNR′R″ in an amount of 5 to 50 moles per 100 moles of RMnX present.

11. Process according to claim 10, characterised in that the reaction is carried out in a THF/sulfolane mixture.

12. New chemical product constituted by a mixed enolate of Mn.

**13.** Product according to claim 12, characterised by the formulae

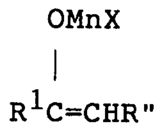

and

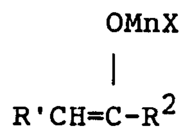

where $R^1$, $R^2$, R′ and R″ are $C_1$ to $C_{17}$ aliphatic hydrocarbon groups and/or arylic groups, X being defined in claim 2.

**14.** Product according to claim 13, characterised in that X is a basic group as defined in one of claims 6 and 7.

**15.** Application of a mixed Mn enolate, according to one of claims 12 to 14, in the preparation of an enol or ketone derivative, characterised in that the mixed Mn enolate is subjected to acylation, to alkylation, to alkenylation, to silylation, to halogenation or to hydroxyalkylation, with elimination of Mn.

**Claims for the following Contracting States : ES, GR**

**1.** Process for preparation of an enolic derivative from a ketone, by the action of an organometallic compound, characterised in that the compound is a mixed organomanganous compound leading to the formation of an enolate of Mn.

**2.** Process according to claim 1, characterised in that in the mixed organomanganous compound R-Mn-X, R denotes a hydrocarbon group, or an amino group or halogen, and X any group able to constitute a ligand of a manganese complex and, in particular, a halogen, an anion derived from S, P, B, C or Si, an oxy- or thio-hydrocarbon group, or an amino group.

**3.** Process according to claim 2, characterised in that R is a $C_1$ to $C_{20}$ alkyl chain, preferably $C_1$ to $C_{12}$, a $C_2$ to $C_{20}$, preferably $C_2$ to $C_{12}$, alkenyl or alkynyl group, or a cycloalkyl, particularly cyclopentyl or cyclohexyl, group, possibly carrying one or more $C_1$ to $C_{12}$ alkyl substituents.

**4.** Process according to claim 3, characterised in that R is methyl.

**5.** Process according to claim 2, characterised in that R is an aryl, in particular phenyl, or naphthyl, possibly carrying 1 to 3 $C_1$ to $C_{12}$ alkyl substituents.

**6.** Process according to one of claims 1 to 5, characterised in that X is a basic group, in particular -NR′R″, where R′ and R″ are the same or different hydrocarbon groups, preferably $C_1$ to $C_{18}$ aliphatic and/or arylic groups possibly being substituted with 1 to 3 $C_1$ to $C_6$ alkyl groups.

**7.** Process according to claim 6, characterised in that X is an -NR′R″ group, where R is a $C_1$ to $C_6$ alkyl; R″ being an aryl group, or else, at the same time, R′ and R″ are aryl groups.

**8.** Process according to one of the preceding claims, in which the reagents are provided in a solvent, more particularly in an ether, most particularly tetrahydrofuran.

**9.** Process according to one of the preceding claims, characterised in that the reaction takes place between -50° and +75°C, particularly between -10° and +60°C.

**10.** Process according to one of the preceding claims, characterised in that, in RMnX, X is a halogen or an anion, and the solution of the reagents is mixed with an amine HNR′R″ in an amount of 5 to 50 moles per 100 moles of RMnX present.

**11.** Process according to claim 10, characterised in that the reaction is carried out in a THF/sulfolane mixture.

**12.** Process according to claim 2, characterised in that the enolate is of the form

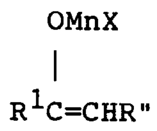

and/or

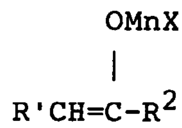

where $R^1$, $R^2$, R′ and R″ are $C_1$ to $C_{17}$ aliphatic hydrocarbon groups and/or arylic groups.

**13.** Process according to one of claims 2 to 11, characterised in that X is a hydrocarbon group, particularly -NR′R″, when R is a halogen.

**14.** Application of a process according to one of claims 1 to 13 in the preparation of an enol or ketone derivative, characterised in that the mixed Mn enolate is subjected to acylation, to alkylation, to alkenylation, to silylation, to halogenation or to hydroxyalkylation, with elimination of Mn.